# EUROPEAN PATENT SPECIFICATION

(11) **EP 1 255 759 B1**
(45) Date of publication and mention of the grant of the patent: **01.08.2007**
(21) Application number: 01910540.2
(22) Date of filing: 09.02.2001
(51) Int. Cl.: C07D 498/18, C07D 491/22, C07F 7/18

(54) **SYNTHETIC PROCESS FOR AN INTERMEDIATE FOR ECTEINASCIDIN AND PHTHALASCIDIN COMPOUNDS**
SYNTHETISCHER PROZESS FÜR EIN INTERMEDIAT VON ECTEINACIDIN UND PHTHALASCIDIN VERBINDUNGEN
PROCEDE DE SYNTHESE D'UN INTERMEDIAIRE DE COMPOSES ECTEINASCIDINE ET PHTHALASCIDINE

(30) Priority: 11.02.2000 US 181795 P
(43) Date of publication of application: 13.11.2002
(73) Proprietor: PRESIDENT AND FELLOWS OF HARVARD COLLEGE, Cambridge, MA 02138-5701 (US)
(72) Inventor: COREY, Elias, J., Cambridge, MA 02140 (US)
(74) Representative: Wakerley, Helen Rachael
(86) International application number: PCT/US2001/004376
(87) International publication number: WO 2001/058905

(56) References cited:
- US-A- 5 721 362
- US-A- 6 124 292
- CUEVAS ET AL.: 'Synthesis of ecteinascidin ET-743 and phthalascidin Pt-650 from cyanosafracin B.' ORGANIC LETTERS vol. 2, no. 16, 19 July 2000, pages 2545 - 2548, XP002940454
- MARTINEZ ET AL.: 'A new, more efficient and effective process for the synthesis of a key pentacyclic intermediate for production of ecteinascidin and phthalascidin antitumor agents' ORGANIC LETTERS vol. 2, no. 7, 15 March 2000, pages 993 - 996, XP002940455
- COREY ET AL.: 'Enantioselective total synthesis of ecteinascidin 743' J. AM. CHEM. SOC. vol. 118, no. 38, 25 September 1996, pages 9202 - 9203, XP002940456
- GREENE ET AL.: 'Protective groups in organic synthesis', 1991, JOHN WILEY & SONS, INC., NEW YORK XP002939992 pages 77 and 83, second edition

## Description

### BACKGROUND OF THE INVENTION

Ecteinascidin 743 (1, Et 743) is an exceedingly potent marine-derived antitumor agent¹ which is now being studied in various clinics with human patients.² Because this compound is not sufficiently available from the natural source, the tunicate *Ecteinascidia turbinata,* it is being produced industrially by the totally synthetic route described in 1996.³ US 5721362 teaches a synthetic process for the production of ecteunascidin compounds and related structures, including saframycins.

More recently a structural analog of Et 743, compound 2 (phthalascidin, Pt 650) has been found to exhibit antitumor activity essentially indistinguishable from 1.⁴

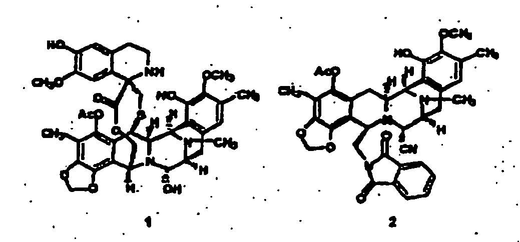

Both 1 and 2 are synthesized from building blocks 3 and 4³ via a common pentacyclic intermediate 5.

Both **1** and **2** are synthesized from building blocks **3** and **4**³ via a common pentacyclic intermediate **5**.

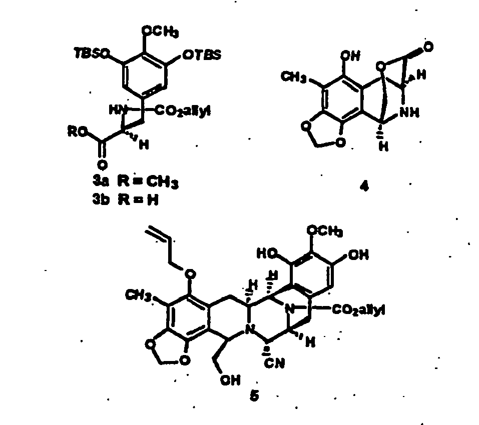

The synthesis of **5** was accomplished originally³ from building blocks **3a** and **4** in six steps with an overall yield of 35% (average yield per step of *ca*. 84%). Because the industrial syntheses of **1** and/or **2** would eventually have to be produced economically on a multi-kilogram scale, we sought to find a more efficient and reproducible alternative route from **2** and **3** to **5.**

### SUMMARY OF THE INVENTION

One embodiment of the present invention is thus directed to a new synthetic process for the preparation of the intermediate compound 5 which is simpler to carry out than the original and which proceeds from **3b³** + **4** to **5** in six steps with an overall yield of 57% (average yield per step of nearly 92%). The preferred process for the synthesis of pentacycle **5** is summarized below in Scheme 1.⁵

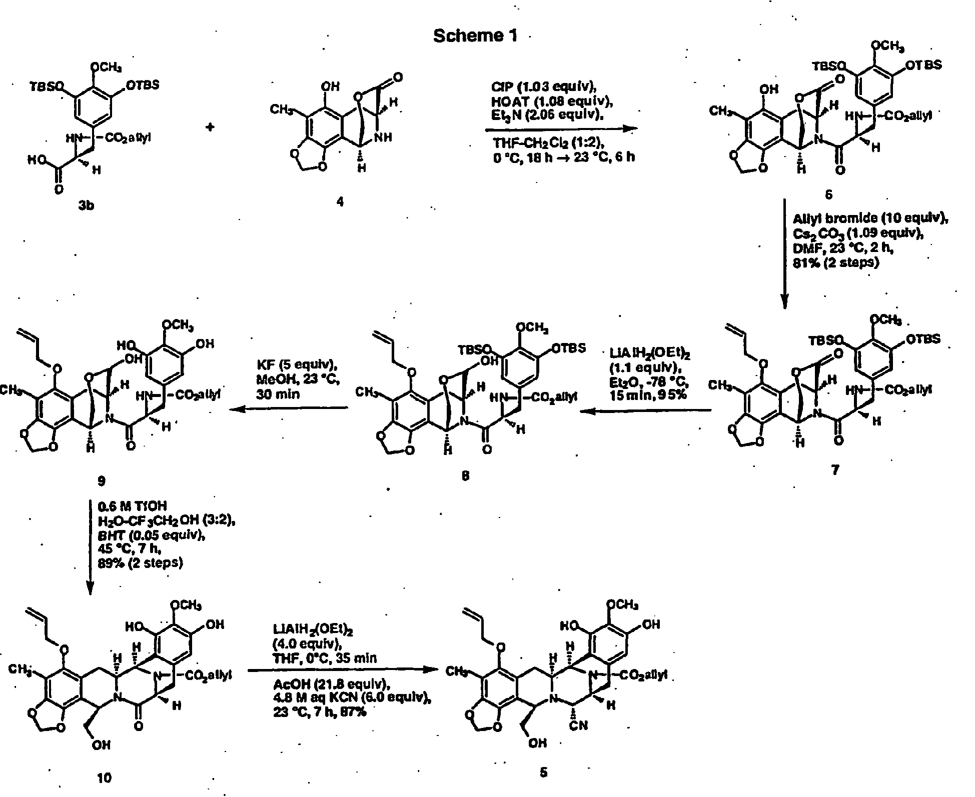

The second preferred embodiment of the present invention entails a new synthetic process for converting the pentacycle compound **5** to phthalascidin **2,** which proceeds smoothly and in excellent yield (average yield per step 90.8%). This process is outlined below in Scheme 2.

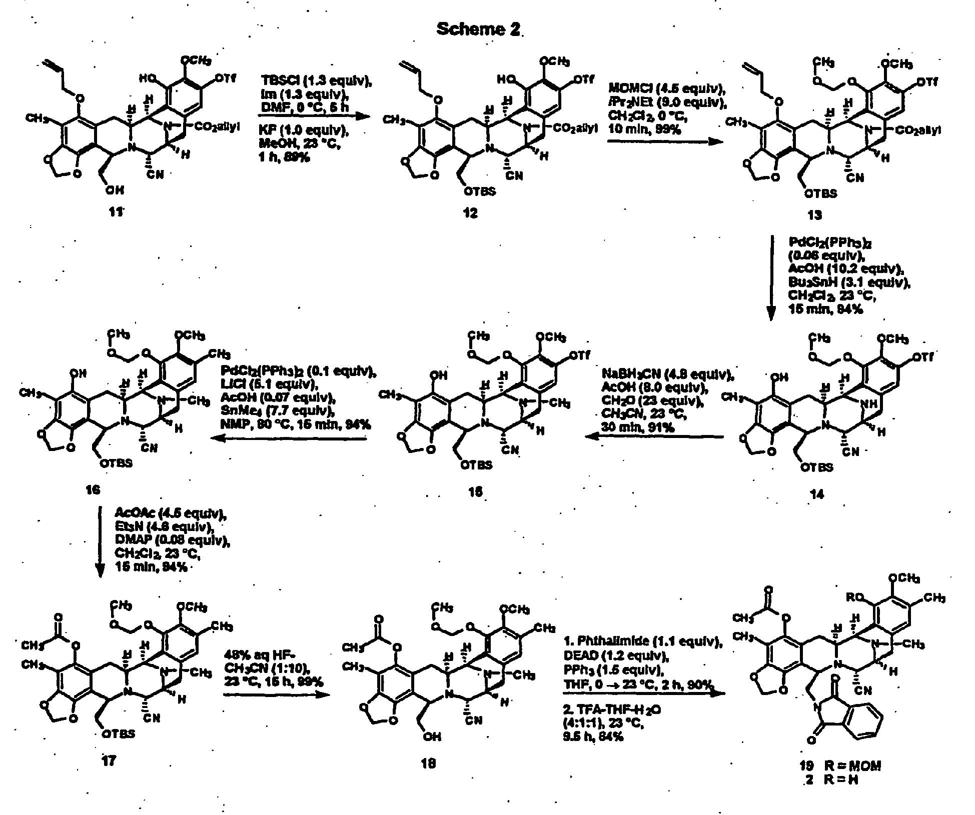

### DETAILED DESCRIPTION OF THE PREFERRED EMBODIMENTS

As illustrated above in Scheme 1, a solution of azeotropically dried (C₇H₈-THF) amino lactone **4** in THF at 0 °C was treated dropwise with an acylating reagent prepared from the acid 3b³ (1.03 equiv), 1-hydroxy-7-azabenzo-triazole (HOAT, 1.08 equiv), 2-chloro-1,3-dimethylimidazolidinium hexafluorophosphate (CIP, 1.03 equiv) and triethylamine (2.06 equiv) in CH₂Cl₂ solution at 0°C.⁶

The coupling product **6**, which was obtained by extractive workup, was allylated without further purification by treatment in DMF solution at 23 °C with excess allyl bromide and 1.09 equiv of Cs₂CO₃ to give amide **7** in 81% overall yield from **3a** and **4** after flash chromatography on silica geL

Selective reduction of the lactone function of **7** to the corresponding lactol (8) was effected by reaction with 1.1 equiv of lithium diethoxyaluminum hydride (LiAlH₂(OEt)₂) in ether at -78° for 15 min in 95% yield.^{7,8} Desilylation of **8** to **9** and cyclization of **9** (without purification) using 0.6 *M* triflic acid in 3:2 H₂O-CF₃CH₂OH at 45 °C for 7 h produced the pentacyclic product **10** in 89% overall yield from **8**.

Finally, the lactam function of **10** could be reduced cleanly by treatment with 4 equiv of LIAlH₂(OEt)₂ in THF at 0 °C for 35 min to the corresponding cyclic aminal which upon exposure to HCN provided the pentacyclic amino nitrile 5 in 87% overall yield from **10** after flash chromatography on silica gel.⁹

The synthesis of 5 which is outlined in Scheme 1 and described above is advantageous relative to the originally used synthetic pathway³ not only because of the substantially greater overall yield (57 vs 35%), but also because of the simplicity and reproducibility of the individual steps, especially the amide coupling (**2a** + **3** → **6**) and the internal Pictet-Spengler cyclization (**9 - 10**). In addition no difficulties have been encountered either in product purification or scale up.

A critical element to the success of the sequence shown in Scheme 1 was the high efficiency and selectivity of LiAlH₂(OEt)₂ for the two reduction steps: **8** → **9** and **10** → **5**, which suggest that this reagent can be used to advantage in synthesis much more frequently than it has been previously.

In Scheme 2, the pentacyclic triol **5** was first converted to the phenolic monotriflate **11** (step not shown) by treatment with 1.1 equiv of PhNTf₂ (McMurry reagent), 2 equiv of Et₃N and 0.2 equiv of 4-dimethyl-aminopyridine in CH₂Cl₂ at - 30 °C for 38 h (74%). Conversion of **11** to the mono *t*-butyldimethylsilyl (TBS) ether **12** and etherification with methoxymethyl chloride (MOMCl) produced **23** in high yield.

Cleavage of the *N*-allyloxycarbonyl and O-allyl groups in **13** gave the secondary amine **14** (94%) which was *N*-methylated to **15** and C-methylated to **16.** Acetylation of phenol **16** produced the corresponding acetate **17** which upon desilylation formed the primary alcohol **18.** Mitsunobu displacement of the primary hydroxyl of **18** produced the phthalimide **19** which upon acid-catalyzed cleavage of the methoxymethyl ether provided pure phthalascidin **2.**

Since the original synthetic route to Et 743 (1) has proved to be acceptable for large scale synthesis, it is our expectation that the improved process described herein will be even more useful, as will the new route to phthalascidin (2).⁴ Because phthalascidin is more stable than ecteinascidin 743 and considerably easier to make, it may prove to be a more practical therapeutic agent.

The present invention will be further illustrated with reference to the following examples which aid in the understanding of the present invention, but which are not to be construed as limitations thereof. All percentages reported herein, unless otherwise specified, are percent by weight. All temperatures are expressed in degrees Celsius.

### Example 1:

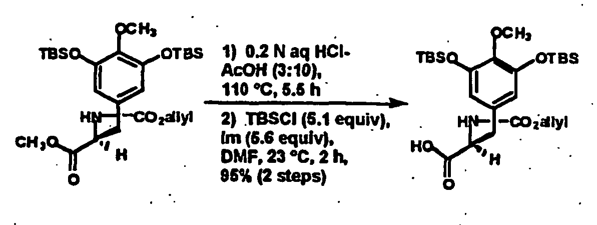

The acid (224 mg, 0.400 mmol) was dissolved in distilled acetic acid (5.0 mL) and 0.2 N HCl¹⁰ (1.5 mL) and heated to 110°C. After 5.5 h, the reaction was concentrated *in vacuo* and dried by repetitive *in vacuo* azeotropic concentration with toluene (3 x 10 mL) and dissolved in DMF (1.0 mL). *tert*-Butyldimethylsilyl chloride (304 mg, 2.03 mmol) and imidazole (152 mg, 2.24 mmol) were added as solids and the mixture was stirred at 23 °C for 2 h. The reaction was quenched with 2:1 acetic acid-water (1.5 mL) and stirred for 30 min. The reaction was poured into 0.5 M aqueous oxalic acid (100 mL) and extracted with 3:7 ethyl acetate-hexane (2 x 100 mL). The combined organic layers were washed with saturated aqueous sodium chloride (100 mL), dried over sodium sulfate, filtered and concentrated in *vacuo.* The residue was purified by flash column, chromatography (100 mL silica gel, gradient 1:1 ethyl acetate-hexane to 0.1% acetic acid-ethyl acetate) to afford the desired product as a substantially pure clear viscous oil (204.6 mg, 95%).

R*_{f}* 0.10 (ethyl acetate); ¹H NMR (400 MHz, CDCl₃) δ 10.25 (br s, 1H), 6.32 (s, 2H), 5.90 (ddt, J=17.0, 10.6, 5.4 Hz, 1H), 5.28 (d, J=17.1 Hz, 1H), 5.20 (d, J=10.4 Hz, 1H), 5.11 (d, J=8.0 Hz, 1H), 4.61-4.57 (m, 1H), 4.55 (d, J=5.5 Hz, 2H), 3.70 (s, 3H), 3.04 (dd, J=14.0, 5.1 Hz, 1H), 2.93 (dd, J=14.0, 6.4 Hz, 1H), 0.99 (s, 18H), 0.15 (s, 12H); ¹³C NMR (101 MHz, CDCl₃) δ 176.3, 155.7, 149.9, 142.2, 132.5, 130.5, 118.0, 115.6, 66.1, 60.0, 54.5, 37.2, 25.8, 18.4, -4.6; FTIR (neat) 3438 (m), 3331 (m), 3088 (m v br), 2956 (s), 2931 (s), 2894 (s), 2863 (s), 1719 (s), 1578 (s), 1496 (s), 1435 (s), 1361 (s), 1253 (s), 1231 (s), 1093 (s), 1010 (m), 938 (w), 831 (s) cm⁻¹; HPLC analysis was performed after derivatization using diazomethane to make the methyl ester (ChiralPak AD, 1% isopropanol in hexane, flow rate: 1.0 mL/min, λ = 226 nm), 96% ee, R*_{T}* = 11.1 min (major), 9.2 min (minor); HRMS (FAB), [m+H]/z calc'd for C₂₆H₄₆O₇NSi₂: 540.2813, found 540.2823; [α]_{D}²³ +18.8° (c 1.0, methylene chloride).

### Example 2:

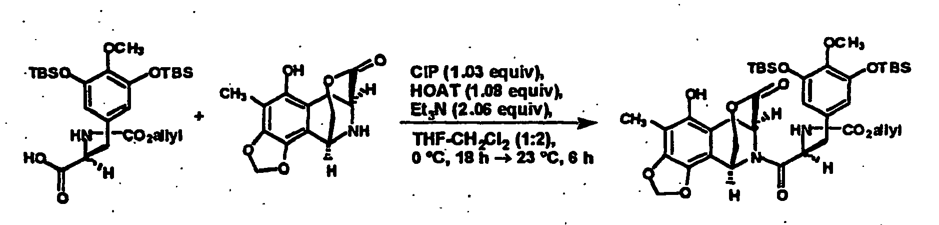

The amine (100.0 mg, 0.380 mmol) was dried by *in vacuo* azeotropic concentration with 2:3 THF-toluene (5 mL) and dissolved in THF (1.5 mL) and cooled to 0 °C. In a different flask, the acid (211.7 mg, 0.392 mmol) and 1-hydroxy-7-azabenzontriazole (55.8 mg, 0.410 mmol) were dried by *in vacuo* azeotropic concentration with 2:3 THF-toluene (5 mL) and dissolved in methylene chloride (1.5 mL). To this flask was added 2-chloro-1,3-dimethylimidazolidinium hexafluorophosphate (109.3 mg, 0.392 mmol) as a solid and triethylamine (109 µL, 0.782 mmol) via syringe to afford a clear dark yellow solution. This mixture was stirred at 23 °C for 3 min and then cooled to 0 °C and cannulated into the flask containing the amine. Methylene chloride (1.5 mL) was used to transfer the remains in the flask. The golden solution was stirred at 0 °C for 18 h, warmed to 23 °C and stirred an additional 6 h. The reaction was diluted with ethyl acetate (6 mL) and partially concentrated *in vacuo* to remove methylene chloride. The solution was poured into 0.5 M aqueous acetic acid (100 mL), extracted with 3:7 ethyl acetate-hexane (100 mL) and washed with saturated aqueous sodium bicarbonate (100 mL). The aqueous layers were re-extracted 3:7 ethyl acetate-hexane (100 mL) and the combined organic layers were dried over sodium sulfate, filtered and concentrated *in vacuo* to afford a clear film (∼300 mg). This residue was used without further purification. The material can be purified by flash column chromatography (100 mL silica gel, gradient 1:3 to 2:3 ethyl acetate-hexane), however only a 50% yield was obtained presumably due to silica gel promoted decomposition.

R*_{f}* 0.36 (2:3 ethyl acetate-hexane); ¹H NMR (400 MHz, CDCl₃) δ (mixture of carbamate and amide retainers) 6.35, (s, 1H), 6.20 (s, 1H), 5.91-5.81 (m, 3H), 5.94-5.59 (m, 1.5H), 5.42 (d, J=3.3 Hz, 0.5H), 5.30-5.03 (m, 3H), 4.74-4.63 (m, 1H), 4.60 (dd, J=10.8,3.1 Hz, 0.5H), 4.53 (br s, 1M), 4.45 (d, J=5.1 Hz, 1H), 4.36 (d, J=10.6 Hz, 0.5H), 4.19 (d, J=10.6 Hz, 0.5H), 3.68 (s, 1.5H), 3.61 (s, 1.5H), 3.56 (d, J=8.4 Hz, 0.5H), 3.03-2.90 (m, 3H), 2.79 (dd, J=13.0, 4.6 Hz, 0.5H), 2.24 (d, J=16.0 Hz, 0.5H), 2.06 (br s, 3H), 0.99 (s, 9H), 0.91 (s, 9H), 0.15 (s, 6H), 0.06 (s, 6H); ¹³C NMR (101 MHz, CDCl₃) δ (mixture of carbamate and amide rotamers) 169.2, 169.0, 167.9, 167.5, 155.6, 155.2, 150.1, 149.7, 146.9, 146.5, 145.1, 145.0, 142.1, 141.7, 136.8, 136.2, 132.4, 132.3, 130.7, 130.3, 117.9, 117.8, 115.5, 115.3, 111.3, 110.9, 110.5, 108.1, 107.8, 101.4, 73.0, 66.1, 65.9, 60.0, 59.9, 54.7, 52.2, 51.9, 51.0, 47.6, 43.2, 39.6, 38.5, 29.1, 27.4, 25.8, 25.7, 18.4, 18.3, 8.9, -4.53, -4.56, -4.65, -4.74; FTIR (neat) 3406 (w br), 3319 (w br), 2956 (m), 2931 (m), 2894 (w), 2856 (m), 1725 (m), 1644 (m), 1575 (m), 1494 (m), 1463 (m), 1431 (s), 1356 (w), 1231 (s), 1163 (w), 1094 (s), 1044 (m), 1013 (m), 831 (s) cm⁻¹; HRMS (ESI), [m+H]/z calc'd for C₃₉H₅₇O₁₁N₂Si₂: 785.3501, found 785.3469; [α]_{D}²⁴ +20.5° (c 1.0, chloroform).

### Example 3:

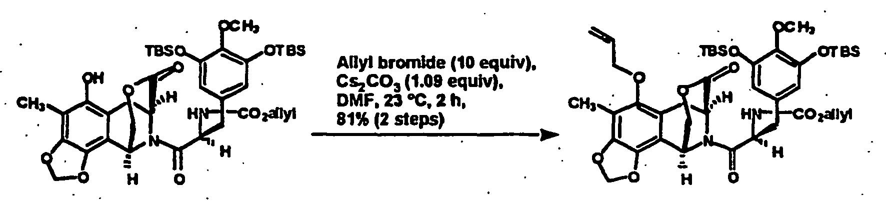

Phenol (~300 mg, 0.380 mmol) was dried by *in vacuo* azeotropic concentration with toluene (5 mL) and dissolved in DMF (15 mL). Allyl bromide (330 µL, 3.82 mmol) was added via syringe and cesium carbonate (134.7 mg, 0.413 mmol), gently flame dried *in vacuo,* was added as a solid and the reaction was stirred at 23 °C for 2 h. The reaction was poured into water (300 mL), extracted with 1:4 ethyl acetate-hexane (2 x 150 mL), washed with saturated aqueous sodium chloride (100 mL), dried over sodium sulfate, filtered and concentrated in *vacuo.* The residue was purified by flash column chromatography (75 mL silica gel, gradient 1:4 to 3:7 ethyl acetate-hexane) to afford the desired product as a substantially pure clear film (252.9 mg, 81% over two steps). This material was also found to be unstable to silica gel and so a rapid chromatography was critical to obtain the observed yield.

R*_{f}* 0.47 (2:3 ethyl acetate-hexane); ¹H NMR (400 MHz, CDCl₃) δ (mixture of carbamate and amide rotamers) 6.35 (s, 1H), 6.20 (s, 1H), 6.03-5.78 (m, 5H), 5.52-5.44 (m, 1.4H), 5.38-5.33 (m, 1H), 5.31-5.13 (m, 3.6H), 4.73-4.59 (m, 1.4H), 4.55 (d, J=5.1 Hz, 1H), 4.48 (d, J=5.1 Hz, 1H), 4.34 (d, J=10.6 Hz, 0.6H), 4.24-4.04 (m, 3H), 3.68 (s, 1.5H), 3.60 (s, 1.5H), 3.54 (d, J=8.8 Hz, 0.4H), 3.15-2.90 (m, 2.6H), 2.77 (dd, J=12.8, 4.8 Hz, 0.6H), 2.34 (m, 0.4H), 2.12 (s, 1.5H), 2.09 (s, 1.5H), 0.99 (s, 9H), 0.92 (s, 9H), 0.16 (s, 6H); 0.07 (s, 3H), 0.05 (s, 3H); ¹³C NMR (101 MHz, CDCl₃) δ (mixture of carbamate and amide rotamers) 169.2, 168.8, 167.4, 167.1, 155.5, 155.1, 150.2, 150.0, 149.8, 145.5, 145.3, 142.2, 141.9, 139.2, 138.8, 133.4, 133.2, 132.4, 130.6, 130.2, 118.3, 118.0, 117.9, 117.8, 117.7, 117.2, 115.5, 115.2, 114.3, 113.9, 111.1, 110.9, 101.8, 101.7, 73.8, 73.7, 72.8, 66.1, 65.9, 60.04, 59.99, 54.9, 52.1, 51.9, 51.1, 47.7, 43.3, 39.8, 38.5, 29.6, 27.9, 25.8, 25.7, 18.4, 18.3, 9.6, 9.4, -4.51, -4.54, -4.6, -4.7; FTIR (neat) 3306 (w br), 2956 (m), 2931 (m), 2898 (m), 2856 (m), 1750 (m), 1719 (m), 1650 (m), 1575 (m), 1494 (m), 1431 (s), 1363 (m), 1250 (m), 1231 (m), 1163 (w), 1094 (s), 1044 (m), 1013 (m), 944 (w), 919 (w), 831 (s) cm⁻¹; HRMS (ESI), [m+H]/z calc'd for C₄₂H₆₁O₁₁N₂Si₂: 825.3814, found 825.3788;[α]_{D}²⁴ +21.7° (c 1.0, chloroform).

### Example 4:

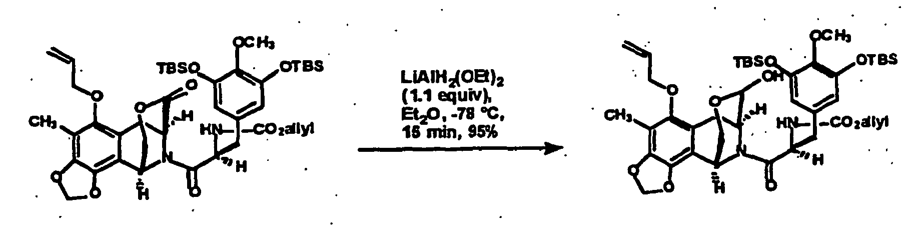

The lactone (354.1 mg, 0.429, mmol) was dried by *in vacuo* azeotropic concentration with toluene (10 mL), dissolved in diethyl ether (8.0 mL) and cooled to -78 °C in a dry ice-acetone bath. A 0.10 M solution of LiAlH₂(OEt)₂ (4.7 mL, 0.47 mmol)¹¹ was added drop-wise down the side of the flask over 2 min. The reaction was stirred at -78 °C for 15 min and then the light yellow solution was poured in 0.1 N HCl (50 mL) at 0 °C while rapidly stirring. This solution was extracted with diethyl ether (2 x 75 mL), washed with saturated aqueous sodium chloride (50 mL), dried over sodium sulfate, filtered and concentrated *in vacuo.* The residue was purified by flash column chromatography (150 mL silica gel, gradient 3:7 to 2:3 to 1:1 ethyl acetate-hexane) to afford the desired product as a substantially pure clear film (339.0 mg, 95%).

*R_{f}* 0.20 (2:3 ethyl acetate-hexane); ¹H NMR (400 MHz, CDCl₃) δ (mixture of anomers, carbamate and amide rotamers) 6.43 (s, 0.2H), 6.37 (s, 0.2H), 6.16 (s, 1.4H), 6.15 (s, 0.2H), 6.05-5.80 (m, 4.4H), 5.82-5.59 (m, 1.2H), 5.41-5.14 (m, 3.8H), 5.07-4.95 (m, 1.5H), 4.85-4.76 (m, 1.6H), 4.61-4.46 (m, 2.2H), 4.26-4.41 (m, 3.8H), 4.10-3.75 (m, 0.5H), 3.68 (s, 0.3H), 3.66 (s, 0.3H),3.63 (s, 2.4H), 3.37 (d, J=11.0 Hz; 0.8H), 3.33-2.94 (m, 0.7H), 2.90-2.65 (m, 2.8H), 2.35 (dd, J=17.7, 7.5 Hz, 0.7H), 2.12 (s, 0.3H), 2.11 (s, 0.3H), 2.09 (s, 2.4H), 1.05 (s, 4.5H), 0.92 (s, 13.5H), 0.16 (s, 3H), 0.07 (s, 4.5H), 0.04 (s, 4.5H); ¹³C NMR (101 MHz, CDCl₃) δ (mixture of anomers, carbamate and amide rotamers) 169.8, 156.1, 149.6, 149.2, 144.6, 141.7, 138.3, 133.8, 132.2, 130.2, 118.9, 118.0, 116.7, 115.1, 113.4, 112.5, 101.3, 93.4, 73.2, 66.2, 62.4, 60.1, 53.6, 51.4, 44.6, 38.5, 26.5, 25.9, 25.8, 18.5, 18.3, 9.5, -4.56, -4.59; FTIR (neat) 3406 (m br), 3325 (m br), 2956 (m), 2931 (m), 2894 (m), 2856 (m), 1714 (m), 1644 (m), 1578 (m), 1496 (m), 1433 (s), 1360 (m), 1255 (m), 1234 (m), 1095 (s), 1044 (m), 1013 (m), 941 (w), 830 (s) cm⁻¹; HRMS (ESI), [m+H]/z calc'd for C₄₂H₆₃O₁₁N₂Si₂: 827.3970, found 827.4009; [α]_{D}²⁵ -1.5° (c 1.0, chloroform).

### Example 5:

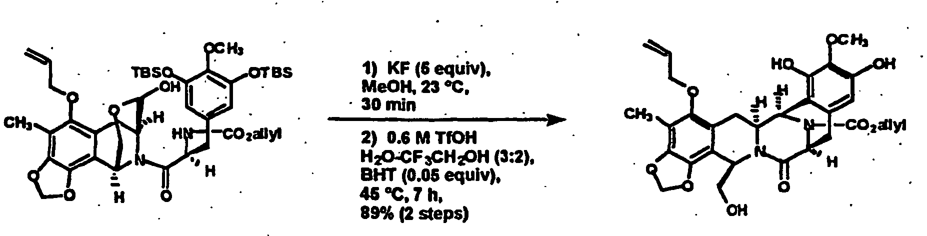

The lactol (316.3 mg, 0.382 mmol) was dissolved in nitrogen purged methanol (3.8 mL). Anhydrous potassium fluoride (110.3 mg, 1.90 mmol) was added as a solid and the vessel was pumped/purged with nitrogen. The reaction was stirred at 23 °C for 30 min and the light pink mixture was diluted with toluene (5 mL) and concentrated *in vacuo*. The residue was dissolved in nitrogen purged 2,2,2-trifluoroethanol (15 mL) and butylated hydroxytoluene (4.3 mg, 0.02 mmol) was added as a solid. The flask was charged with 1.0 M aqueous trifluoromethanesulfonic acid¹² (23 mL) and the vessel was again pumped/purged with nitrogen. The solution was stirred at 45 °C in an oil bath for 7 h. The mixture was partially concentrated *in vacuo,* to remove the alcohol, and poured into 80% saturated aqueous sodium chloride (100 mL), extracted with ethyl acetate (2 x 100 mL), washed with saturated aqueous sodium chloride (50 mL), dried over sodium sulfate, filtered and concentrated *in vacuo.* The residue was purified by flash column chromatography (100 mL silica gel, 5:95 methanol-methylene chloride) to afford the desired product as a substantially pure white solid (198.5 mg, 89%). Crystals were obtained from toluene.

M.p.: 130 ° C (dec.); R*_{f}* 0.11 (5:95 methanol-methylene chloride); ¹H NMR (400 MHz, Acetone-d₆) δ (mixture of carbamate rotamers) 8.34 (br s, 1H), 8.32 (br s, 1H), 6.31 (d, J=4.4 Hz, 1H), 6.14 (m, 1H), 5.97 (s, 1H), 5.97-5.90 (m, 1H), 5.90 (s, 1H), 5.68 (m, 1H), 5.42-5.37 (m, 2H), 5.31-5.22 (m, 2H), 5.18-5.12.(m, 1H), 4.85 (d, J=6.6 Hz, 1H), 4.65-4.55 (m, 2H), 4.38-4.34 (m, 1H), 4.26-4.22 (m, 1H), 3.89-3.86 (m, 1H), 3.77 (s, 3H), 3.71 (m, 1H), 3.57 (d, J=15.0 Hz, 1H), 3.48-3.43 (m, 1H), 3.25-3.13 (m, 2H), 3.00 (d, J=16.8 Hz, 1H), 2.34 (m, 1H), 2.11 (s, 3H); ¹³C NMR (101 MHz, Acetone-d₆) δ (mixture of carbamate rotamers) 169.4, 169.2, 153.8, 153.7, 150.6, 149.3, 148.2, 148.0, 145.5, 141.0, 135.1, 134.5, 133.9, 130.2, 130.1, 122.4, 117.9, 117.8, 117.7, 117.5, 114.2, 112.7, 111.0, 110.8, 108.4, 108.3, 102.1, 75.4, 66.74, 66.69, 65.6, 61.6, 61.2, 60.9, 54.3, 53.5, 52.9, 50.1, 49.3, 34.1, 33.6, 27.5, 9.7; FTIR (KBr) 3400 (s br), 2944 (m), 2881 (m), 1700 (s), 1639 (s), 1501 (w), 1463 (s), 1435 (s), 1356 (m), 1320 (m), 1288 (m), 1269 (m), 1238 (m), 1213 (m), 1166 (m), 1102 (s), 1065 (s), 1030 (m), 999 (m), 938 (m), 807 (w) cm⁻¹; HRMS (ESI), [m+H]/z calc'd for C₃₃H₃₃O₁₀N₂: 581.2135, found 581.2112; [α]_{D}²⁵-27.2°(*c* 0.50, methanol).

### Example 6:

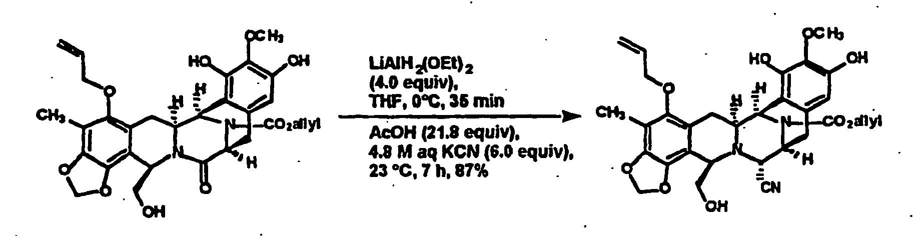

The amide (198.0 mg, 0.341 mmol) was dried by *in vacuo* azeotropic concentration with toluene. (10 mL), dissolved in THF. (10 mL) and cooled to 0 °C. A 0.20 M solution of LiAHl₂(OEt)₂ (6.8 mL, 1.36 mmol)¹³ was added drop-wise over 10 min. The reaction was stirred at 0 °C for 35 min at afford the carbinolamine, R_{f} 0.59 (4:1 ethyl acetate-hexane). Acetic acid (425 µL, 7.44 mmol) was added first in order to quench the reaction. Then 4.8 M aqueous potassium cyanide (425 µL, 2.04 mmol), anhydrous sodium sulfate (2.5 g, 17.6 mmol) and Celite^{®} (6 mL) were added to affect the conversion to the amino-nitrile and to precipitate the aluminum salts. Bubbling was observed and after 5 min the reaction was warmed to 23 °C and stirred for 7 h. The suspension was filtered through a pad of Celite^{®}, eluting with ethyl acetate (100 mL). This solution was concentrated *in vacuo* and purified by flash column chromatography (100 mL silica gel, 2:1 ethyl acetate-hexane) to afford the desired product as a substantially pure white foam (175.6 mg, 87%).

R*_{f}* 0.31 (4:1 ethyl acetate-hexane); ¹H NMR (400 MHz, CDCl₃) δ (mixture of carbamate rotamers) 6.43 (br s, 0.6H), 6.26 (s, 0.4H), 6.24 (s, 0.6H), 6.20 (s, 0.4H), 6.07-6.00 (m, 1H), 5:97-5.82 (m, 4H), 5.61 (s, 0.6H), 5.52 (s, 0.4H), 5.37-5.17 (m, 3H), 4.90 (d, J=7.8 Hz, 0.4H), 4.84 (d, J=8.3 Hz, 0.6H), 4.73-4.60 (m, 2H), 4.16-4.08 (m, 2.6H), 3.97-3.94 (m, 1.4H), 3.77 (s, 1.2H), 3.68-3.61 (m, 1H), 3.62 (s, 1.8H), 3.49-3.36 (m, 1H), 3.29-3.19 (m, 3H), 2.76-2.69 (m, 1H), 2.11 (s, 1.8H), 2.08 (s, 1.2H), 2.00-1.83 (m, 2H); ¹³C NMR (101 MHz, CDCl₃) δ (mixture of carbamate rotamers) 154.3, 153.8, 148.4, 148.34, 1,48.26, 146.2, 145.9, 144.3, 138.8, 133.62, 133.56, 132.7, 132.2, 130.7, 130.3, 120.5, 120.3, 117.9, 117.8, 117.4, 117.2, 116.3, 112.6, 112.5, 112.1, 111.9, 107.2, 106.4, 101.1, 74.5, 74.0, 66.7, 66.5, 64.5, 64.3, 60.8, 60.5, 59.1, 58.9, 58.0, 56.7, 56.6, 49.9, 49,4, 48.9, 48.7, 31.2, 30.5, 29.7, 25.9, 9.43, 9.35; FTIR (neat) 3369 (m br), 2931 (m br), 1688 (m), 1500 (w), 1463 (m) 1431 (s), 1375 (m), 1325 (m), 1294 (m), 1269 (m), 1106 (s), 1063 (m), 994 (m), 956 (w) cm⁻¹; HRMS (ESI), [m+H]/z calc'd for C₃₁H₃₄O₉N₃: 592.2295, found 592.2316; [α]_{D}²⁵ +30.4° (c 1.0, chloroform).

### Example 7:

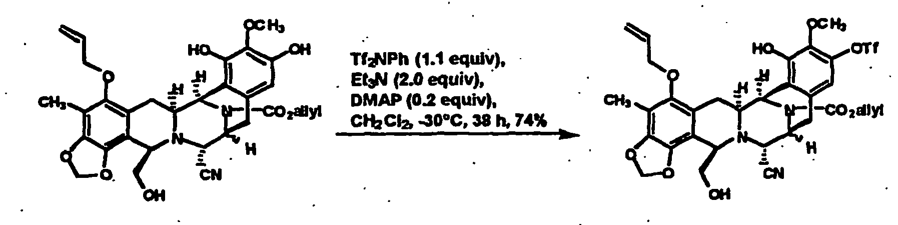

The phenol (170.0 mg, 0.287 mmol) was dried by *in vacuo* azeotropic concentration with toluene (10 mL) and dissolved in methylene chloride (3.0 mL). Triethylamine (80 µL, 0.574 mmol) and 4-dimethylaminopyridine (7.0 mg, 0.0574 mmol) were added and the solution was cooled to -30 °C in a dry ice-acetonitrile bath. *N*-Phenyltrifluoromethanesulfonimide (113.5 mg, 0.318 mmol) was added as a solid and the reaction was stirred at -30 °C in a Cryobath^{®} for 38 h. The mixture was poured into 1:1 saturated aqueous sodium bicarbonate-saturated aqueous sodium chloride (100 mL), extracted with methylene chloride (2 x 75 mL), dried over sodium sulfate, filtered and concentrated *in vacuo.* The residue was purified by flash column chromatography (100 mL silica gel, gradient 2:3 to 3:4 ethyl acetate-hexane) to afford the desired product as a substantially pure clear film (153.4 mg, 74%).

R*_{f}* 0.18 (2:3. ethyl acetate-hexane); ¹H NMR (400 MHz, CDCl₃) δ (mixture of carbamate rotamers) 7.16 (s, 0.6H), 6.63 (s, 0.4H), 6.60 (s, 0.6H), 6.45 (s, 0.4H), 6.08-5.86 (m, 4H), 5.74 (m, 0.6H), 5.59 (m, 0.4H), 5.40-5.16 (m, 4H), 4.96-4.89 (m, 1H), 4.74-4.60 (m, 3H), 4.26 (m, 1H), 4.19-4.15 (m, 2H), 4.00 (m, 1H), 3.89 (s, 1.2H), 3.83 (s, 1.8H), 3.66-3.64 (m, 1H), 3.39-3.24 (m, 4H), 2.91-2.83 (m, 1H), 2.11 (s, 1.2H), 2.05 (s, 1.8H), 1.86-1.78 (m, 1H); ¹³C NMR (101 MHz, CDCl₃) δ (mixture of carbamate rotamers) 154.0, 153.9, 148.6, 148.4, 147.3, 146.6, 144.7, 144.5, 141.3, 141.0, 139.1, 138.9, 136.9, 136.7, 133.7, 132.2, 132.1, 131.6, 129.4, 127.0, 123.0, 121.5, 121.3, 119.9, 118.5 (q, J=321 Hz, CF₃), 118.2, 117.7, 117.6, 117.4, 116.3, 116.1, 112.6, 112.3, 112.1, 112.0, 101.3, 101.2, 74.5, 66.9, 66.7, 65.7, 65.5, 62.0, 61.9, 59.54, 59.48, 58.6, 56.5, 49.8, 49.3, 49.0, 48.4, 31.0, 30.4, 26.1, 26.0, 9.5, 9.4; ¹⁹F NMR (376 MHz, BF₃•OEt₂ standard set at -153.0 ppm, CDCl₃) δ (mixture of carbamate rotamers) -74.02, -74.01; FTIR (neat) 3325 (w br), 2949 (w br), 1688 (m), 1588 (w), 1500 (m), 1425 (s), 1319 (m), 1288 (m), 1256 (m), 1213 (s), 1138 (s), 1106 (m), 1038 (m), 988 (m), 875 (w) cm⁻¹; HRMS (ESI), [m+H]/z calc'd for C₃₂H₃₃O₁₁N₃SF₃: 724.1788, found 724.1803; [α]_{D}²⁶ +34.3° (*c* 1.0, chloroform).

### FOOTNOTES:

The following publications provide background information and are hereby incorporated herein by reference.
(1) The pioneering research in this area is due to Prof. Kenneth L. Rinehart and his group. See, (a) Rinehart, K. L.; Shield, L. S. in Topics in Pharmaceutical Sciences, eds. Breimer, D. D.; Crommelin, D. J. A.; Midha, K. K. (Amsterdam Medical Press, Noordwijk, The Netherlands), 1989, pp. 613. (b) Rinehart, K. L.; Holt, T. G.; Fregeau, N. L.; Keifer, P. A.; Wilson, G. R.; Perun, T. J., Jr.; Sakai, R.; Thompson, A. G.; Stroh, J. G.; Shield, L. S.; Seigler, D. S.; Li, L. H.; Martin, D. G.; Grimmelikhuijzen, C. J. P.; Gäde, G. J. Nat. Prod. 1990, 53, 771. (c) Rinehart, K. L.; Sakai, R; Holt, T.G.; Fregeau, N. L.; Perun, T. J., Jr.; Seigler, D. S.; Wilson, G. R.; Shield, L. S. Pure Appl. Chem. 1990, 62, 1277. (d) Rinehart, K. L.; Holt, T. G.; Fregeau, N. L.; Stroh, J. G.; Keifer, P. A.; Sun, F.; Li, L. H.; Martin, D. G. J. Org. Chem. 1990, 55, 4512. (e) Wright, A. E.; Forleo, D. A.; Gunawardana, G. P.; Gunasekera, S. P.; Koehn, F. E.; McConnell, O. J. J. Org. Chem. 1990, 55, 4508. (f) Sakai, R.; Rinehart, K. L.; Guan, Y.; Wang, H. J. Proc. Natl. Acad. Sci USA 1992, 89, 11456.
(2) (a) Business Week, 13 September 1999, p. 22. (b) Science 1994, 266,1324.
(3) Corey, E. J.; Gin, D. Y.; Kania, R. J. Am. Chem. Soc. 1996, 118, 9202.
(4) Martinez, E. J.; Owa, T.; Schreiber, S. L.; Corey, E. J. Proc. Natl. Acad. Sci. USA 1999, 96, 3496.
(5) See Myers, A. G.; Kung, D. W. J. Am. Chem. Soc. 1999, 121, 10828 for a different approach to the synthesis of structures such as 5.
(6) For carboxylic acid-amine coupling methodology using CIP, see: (a) Akaji, K.; Kuriyama, N.; Kimura, T.; Fujiwara, Y.; Kiso, Y. Tetrahedron Lett. 1992, 33, 3177. (b) Akaji, K.; Kuriyama, N.; Kiso, Y. Tetrahedron Lett. 1994, 35, 3315. (c) Akaji, K.; Kuriyama, N.; Kiso, Y. J. Org. Chem. 1996, 61, 3350.
(7) The reagent LiAlH₂(OEt)₂ was prepared by the addition of a 1.0 M solution of LiAlH₄ in ether to a solution of 1 equiv of ethyl acetate at 0 °C and stirring at 0 °C for 2 h just before use; see: Brown, H. C.; Tsukamoto, A. J. Am Chem Soc. 1964, 86, 1089.
(8) For general reviews on reduction of lactones see: (a) Brown, H. C.; Krishnamurthy, S. Tetrahedron, 1979, 35, 567. (b) Cha, J.S. Org. Prep. Proc. Int, 1989, 21(4), 451. (c) Seyden-Penne, J. Reduction by the Alumino- and Borohydrides in Organic Synthesis; 2nd Ed.; Wiley-VCH: New York, 1997; Section 3.2.5.
(9) For general references on amide reduction by hydride reagents see ref. 7 and also Myers, A. G.; Yang, B. H.; Chen, H.; Gleason, J. L. J. Am. Chem. Soc. 1994, 116, 9361.
(10) Made from nitrogen purged water.
(11) This reagent was made by adding a 1.0 M solution of lithium aluminum hydride in Et₂O (1 equiv) to a solution of ethyl acetate (1 equiv) in Et₂O at 0 ° C. The mixture was stirred at 0 °C for 2 h and a portion of this reagent was used for the reduction of the lactol. Brown, H. C.; Tsukamoto, A. J. Am. Chem. Soc. 1964, 86, 1089.
(12) Made from nitrogen purged water.
(13) See the reference cited in footnote 11.

The present invention has been described in detail, including the preferred embodiments thereof. However, it will be appreciated that those skilled in the art, upon consideration of the present disclosure, may make modifications and/or improvements on this invention and still be within the scope and spirit of this invention as set forth in the following claims.

## Claims

1. A process to prepare an intermediate of ecteinascidin 743, comprising the steps of:
(a) combining the amino lactone 4: 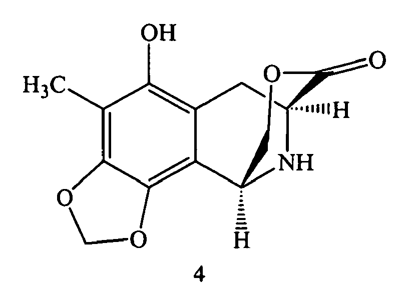 with an acylating reagent prepared from the compound 3b: 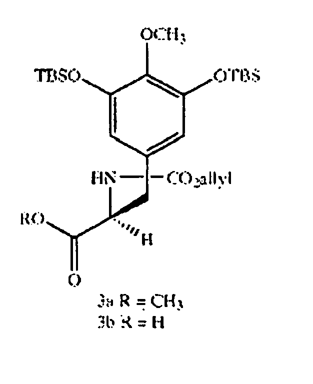 to form the coupled product 6: 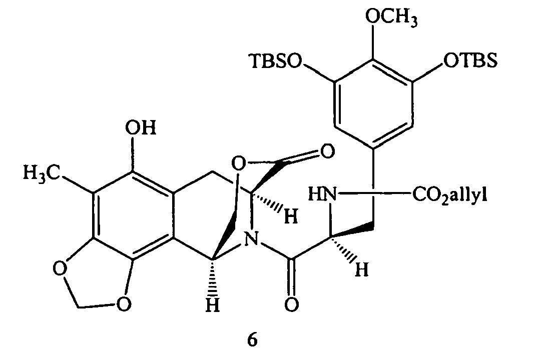
(b) treating compound 6 with an allyl bromide to give amide 7: 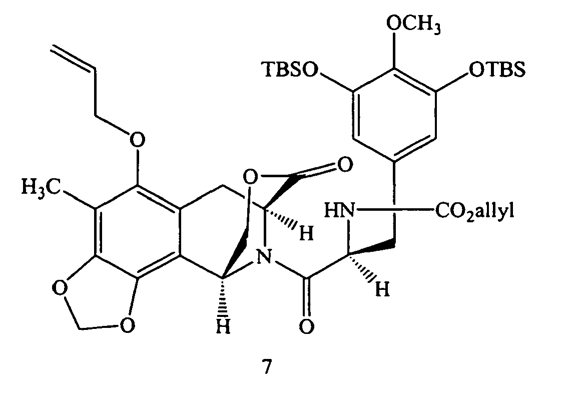
(c) reduction of the lactone compound 7 to the corresponding lactol, compound 8: 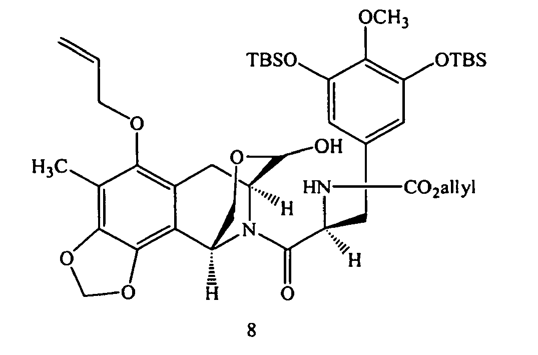
(d) desilylation of compound 8 to compound 9: 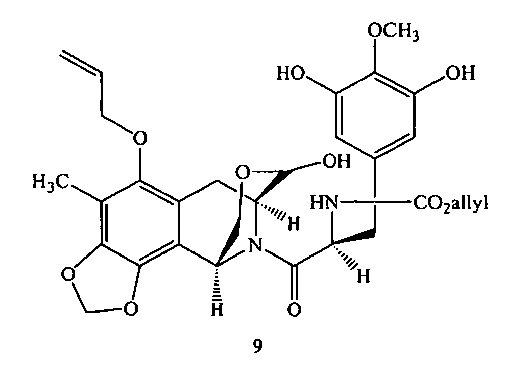
(e) cyclization of compound 9 to compound 10: 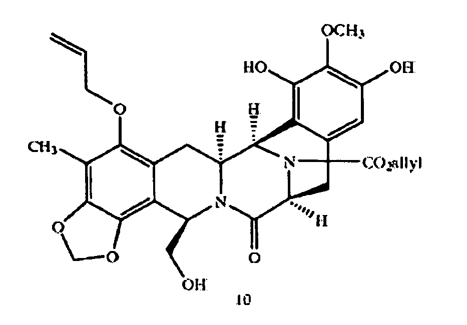
(f) reduction of the lactam in compound 10 to the corresponding cyclic aminal which upon exposure to HCN provided the pentacyclic amino nitrile, compound 5:

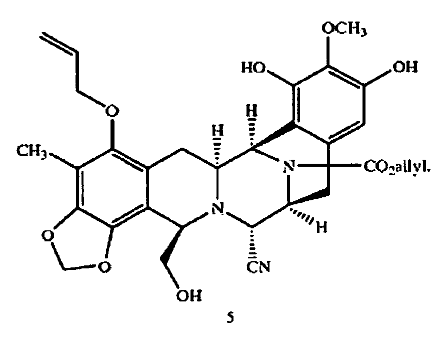

2. The process of claim 1, to prepare phthalascidin, further comprising the steps of
(g) the conversion of the compound of formula 5 to the compound of formula 11 by treatment with PhNTf₂ 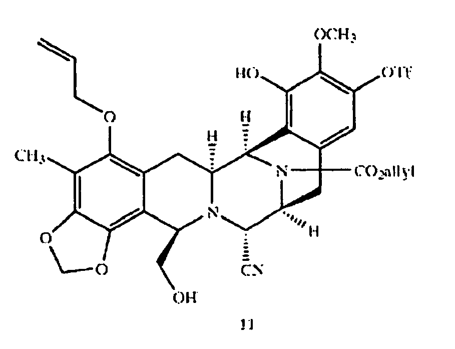
(h) conversion of the compound 11 to the mono *t*-butyldimethylsilyl (TBS) ether, compound 12: 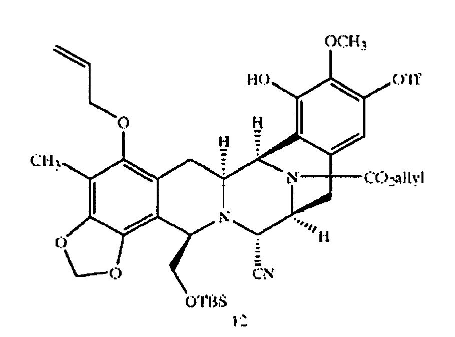
(i) etherification with methoxymethyl chloride to yield compound 13: 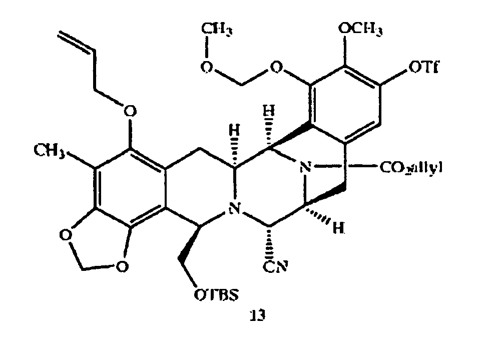
(j) cleaving the N-allyloxycarbonyl and O-allyl groups in 13 gives the secondary amine, compound 14: 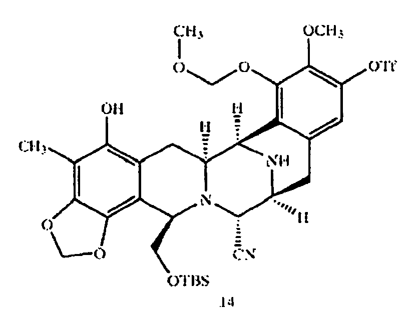
(k) N-methylation of compound 14 affords compound 15: 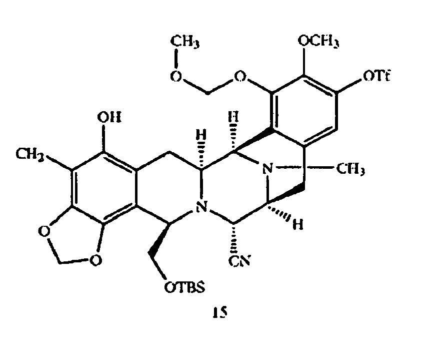
(l) C-methylation of compound 15 affords compound 16: 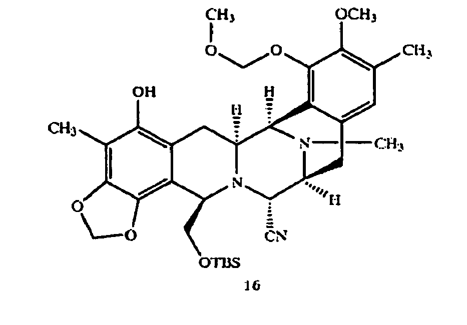
(m) acetylation of phenol 16 affords the corresponding acetate, compound 17: 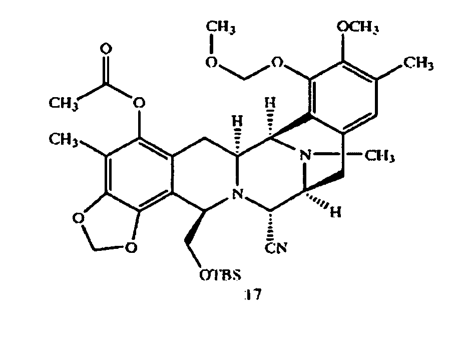
(n) desilylation of compound 17 yields the primary alcohol, compound 18: 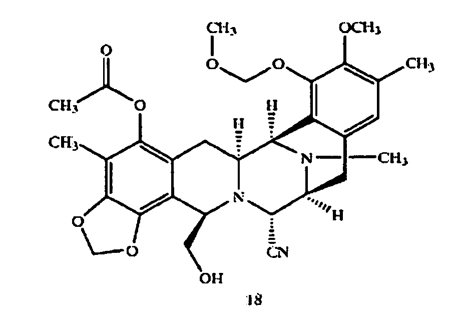
(o) Mitsunobu displacement of the primary hydroxyl of 18, yields the phthalimide, compound 19: 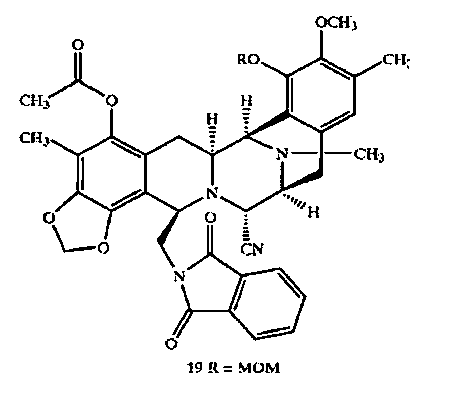
(p) acid-catalyzed cleavage of the methoxymethyl ether in compound 19, generates compound 2 - phthalascidin:

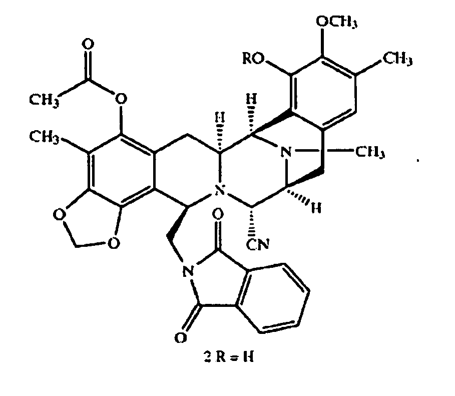

3. The synthetic intermediate of the process of Claim 1 - compound 6:

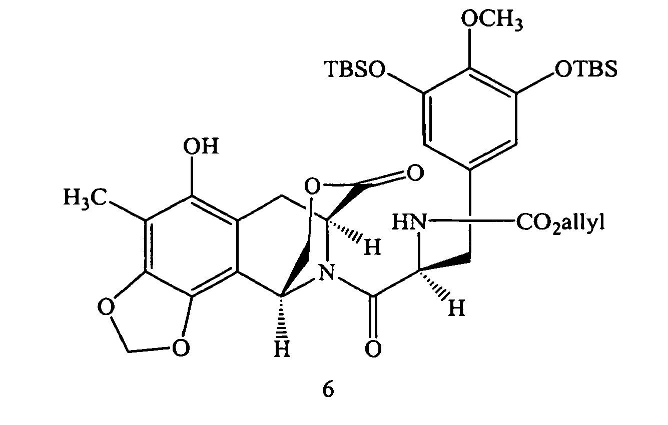

4. The synthetic intermediate of the process of Claim 1 - compound 7:

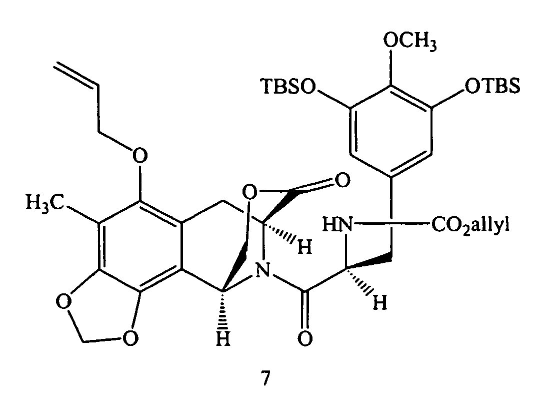

5. The synthetic intermediate of the process of Claim 1 - compound 8:

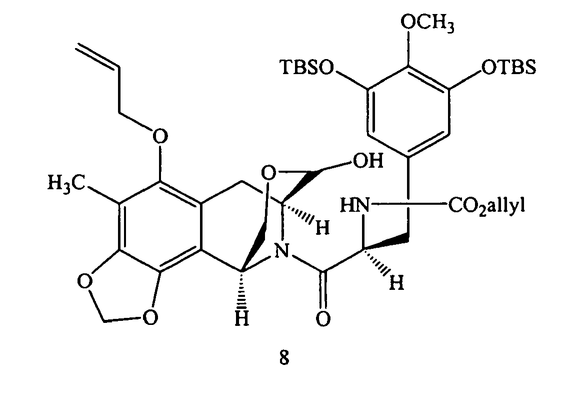

6. The synthetic intermediate of the process of Claim 1 - compound 9:

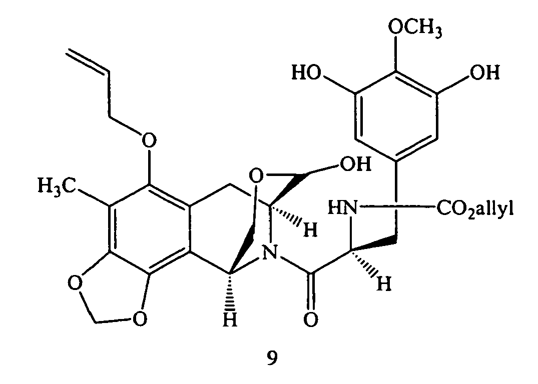

7. The synthetic intermediate of the process of Claim 1 - compound 10:

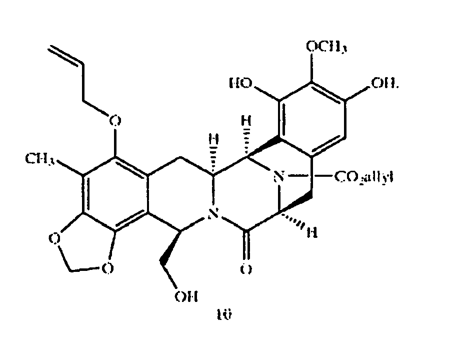

8. The synthetic intermediate of the process of Claim 2 - compound 12:

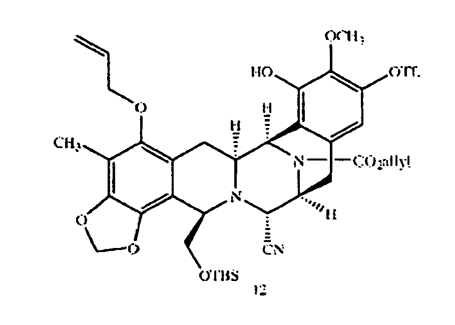

9. The synthetic intermediate of the process of Claim 2 - compound 13:

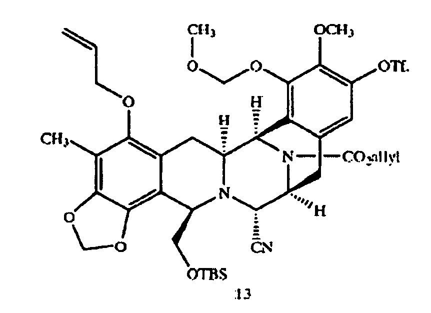

10. The synthetic intermediate of the process of Claim 2 - compound 14:

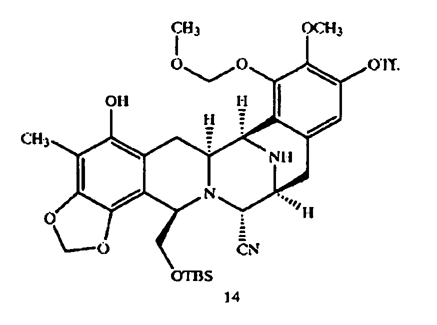

11. The synthetic intermediate of the process of Claim 2 - compound 15:

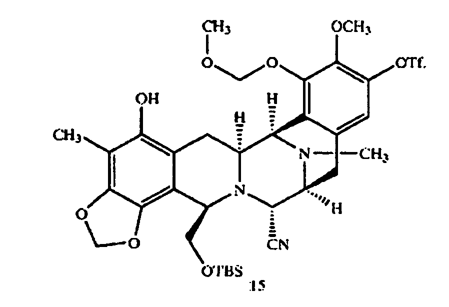

12. The synthetic intermediate of the process of Claim 2 - compound 16:

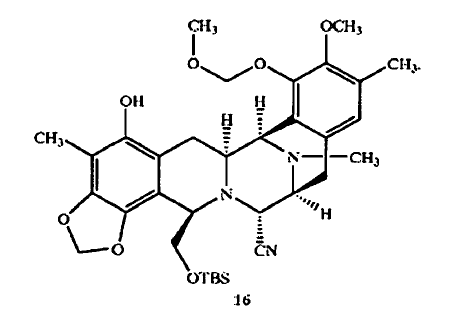

13. The synthetic intermediate of the process of Claim 2 - compound 17:

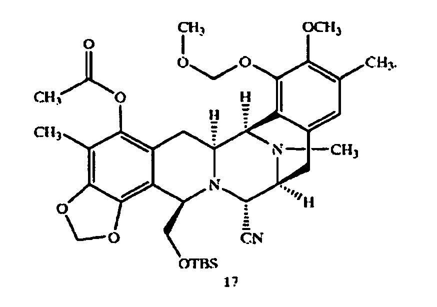

14. The synthetic intermediate of the process of Claim 2 -compound 18:

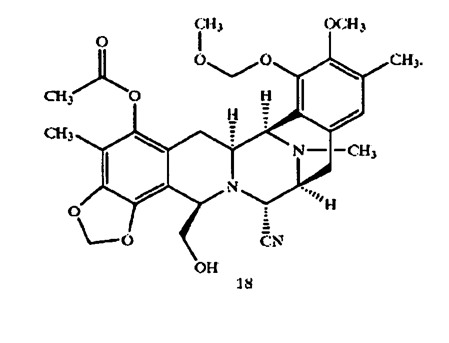

15. The synthetic intermediate of the process of Claim 2 - compound 19:

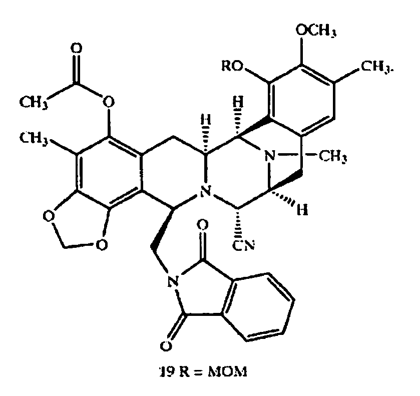

## Patentansprüche

1. Verfahren zur Herstellung eines Intermediats von Ecteinascidin 743, das die folgenden Schritte beinhaltet:
(a) Kombinieren des Aminolactons 4: 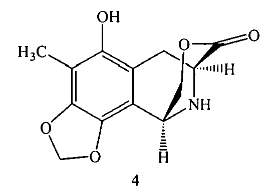 mit einem Acylierungsmittel, hergestellt von Verbindung 3b: 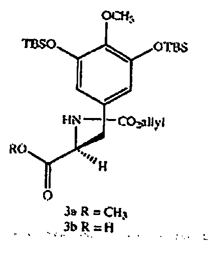 zum Bilden des gekoppelten Produkts 6: 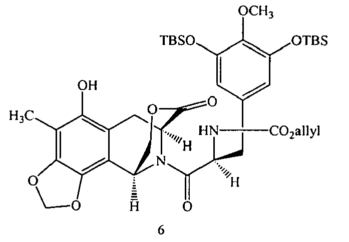
(b) Behandeln von Verbindung 6 mit einem Allylbromid zum Erhalten von Amid 7: 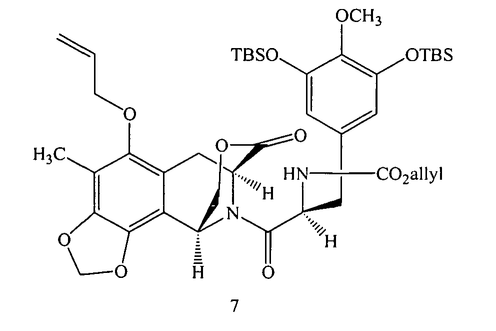
(c) Reduzieren der Lactonverbindung 7 auf das entsprechende Lactol, Verbindung 8: 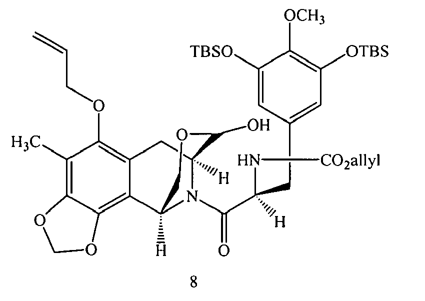
(d) Desilylieren von Verbindung 8 zu Verbindung 9: 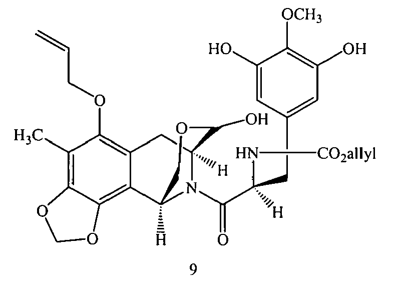
(e) Cyclisieren von Verbindung 9 zu Verbindung 10: 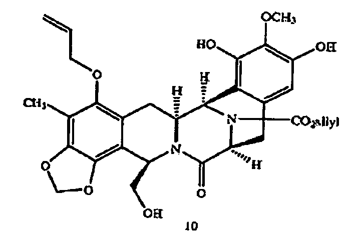
(f) Reduzieren des Lactams in Verbindung 10 auf das entsprechende cyclische Aminal, das nach Kontakt mit HCN das pentacyclische Aminonitril, Verbindung 5, ergibt:

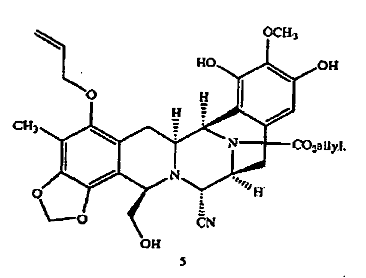

2. Verfahren nach Anspruch 1 zur Herstellung von Phthalascidin, das ferner die folgenden Schritte beinhaltet:
(g) Umwandeln der Verbindung von Formel 5 in die Verbindung von Formel 11 durch Behandlung mit PhNTf₂: 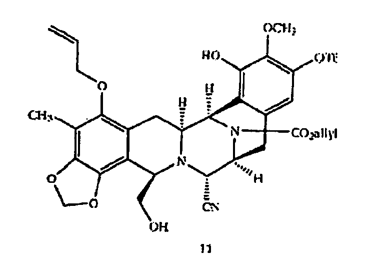
(h) Umwandeln der Verbindung 11 in das Mono-t-butyldimethylsilyl-(TBS)-Ether, Verbindung 12: 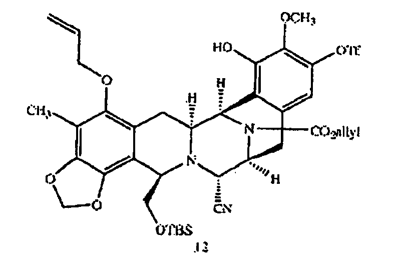
(i) Verethern mit Methoxymethylchlorid zum Erhalten von Verbindung 13: 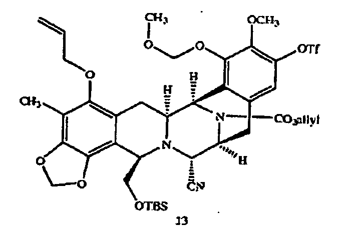
(j) Spalten der N-Allyloxycarbonyl- und O-Allylgruppen in 13 zum Erhalten des sekundären Amins, Verbindung 14: 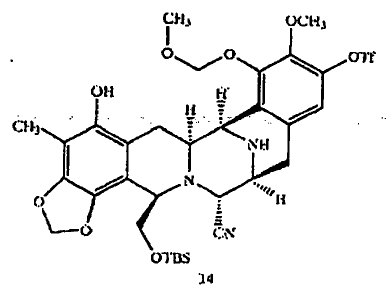
(k) N-Methylieren von Verbindung 14 zum Erhalten von Verbindung 15: 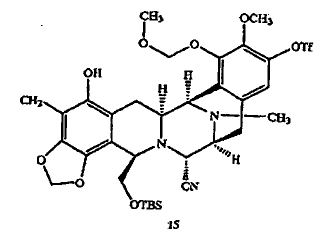
(l) C-Methylieren von Verbindung 15 zum Erhalten von Verbindung 16: 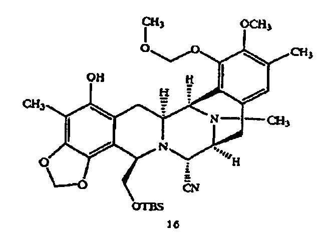
(m) Acetylieren von Phenol 16 zum Erhalten des entsprechenden Acetats, Verbindung 17: 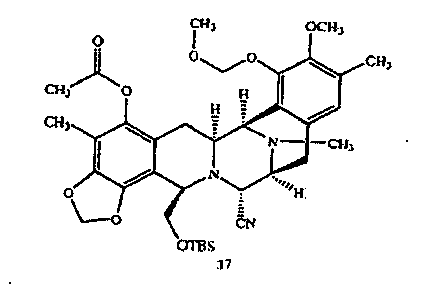
(n) Desilylieren von Verbindung 17 zum Erhalten des primären Alkohols, Verbindung 18: 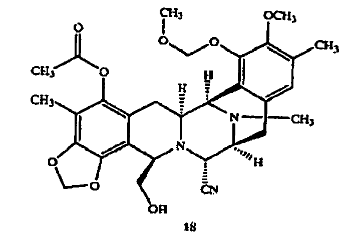
(o) Verdrängen des primären Hydroxyls von 18 nach Mitsunobu zum Erhalten des Phthalimids, Verbindung 19: 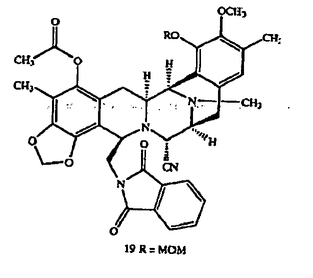
(p) säurekatalysiertes Spalten des Methoxymethylethers in Verbindung 19 zum Erzeugen von Verbindung 2, Phthalascidin:

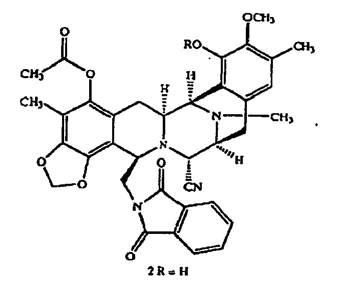

3. Synthetisches Intermediat des Verfahrens nach Anspruch 1 - Verbindung 6:

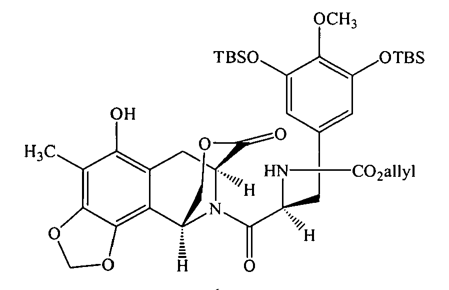

4. Synthetisches Intermediat des Verfahrens nach Anspruch 1 - Verbindung 7:

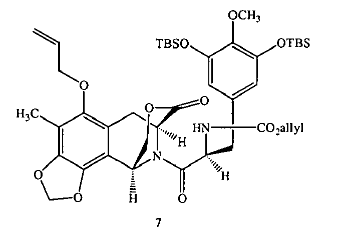

5. Synthetisches Intermediat des Verfahrens nach Anspruch 1 - Verbindung 8:

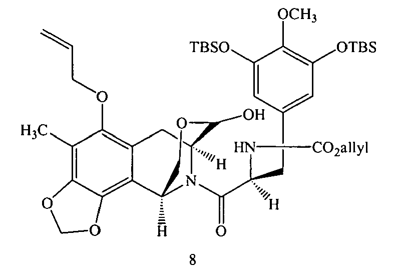

6. Synthetisches Intermediat des Verfahrens nach Anspruch 1 - Verbindung 9:

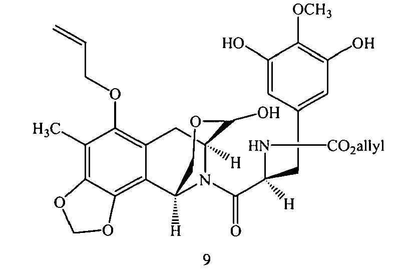

7. Synthetisches Intermediat des Verfahrens nach Anspruch 1 - Verbindung 10:

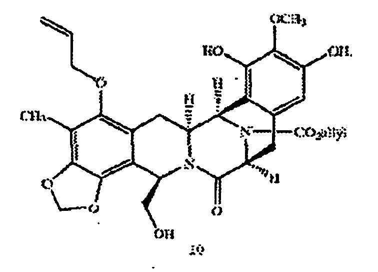

8. Synthetisches Intermediat des Verfahrens nach Anspruch 2 - Verbindung 12:

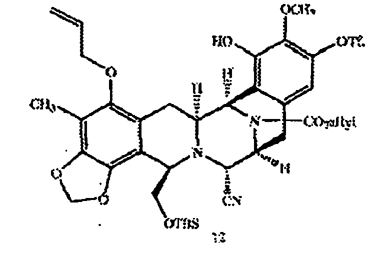

9. Synthetisches Intermediat des Verfahrens nach Anspruch 2 - Verbindung 13:

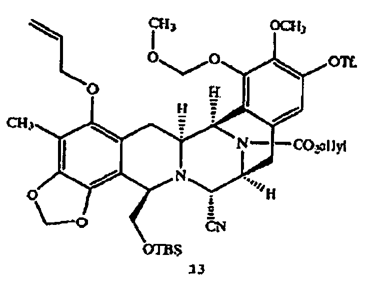

10. Synthetisches Intermediat des Verfahrens nach Anspruch 2 - Verbindung 14:

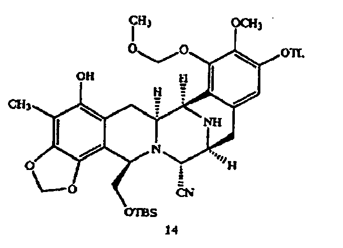

11. Synthetisches Intermediat des Verfahrens nach Anspruch 2 - Verbindung 15:

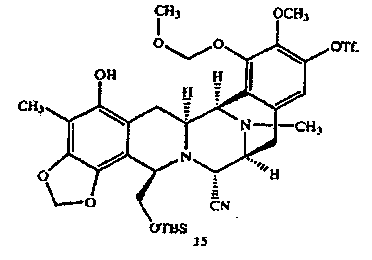

12. Synthetisches Intermediat des Verfahrens nach Anspruch 2 - Verbindung 16:

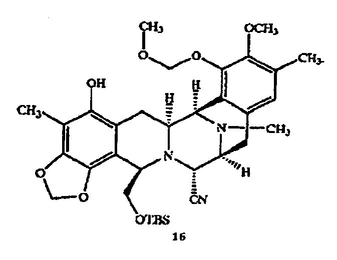

13. Synthetisches Intermediat des Verfahrens nach Anspruch 2 - Verbindung 17:

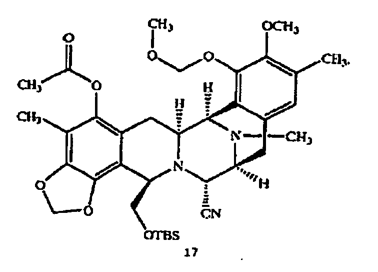

14. Synthetisches Intermediat des Verfahrens nach Anspruch 2 - Verbindung 18:

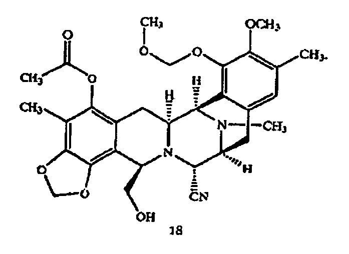

15. Synthetisches Intermediat des Verfahrens nach Anspruch 2 - Verbindung 19:

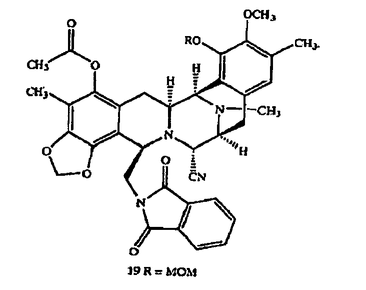

## Revendications

1. Procédé pour la préparation d'un intermédiaire de l'extéinascidine 743, comprenant les étapes consistant à :
(a) combiner l'amino-lactone 4: 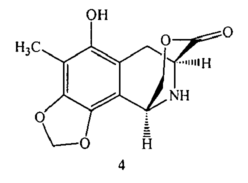 avec un réactif d'acylation préparé avec le composé 3b : 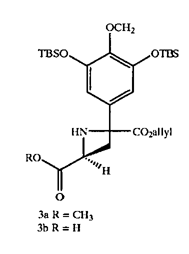 pour former le produit couplé 6 : 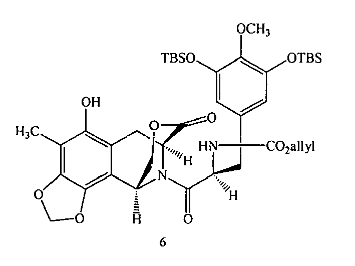
(b) traiter le composé 6 avec un bromure d'allyle pour donner l'amide 7 : 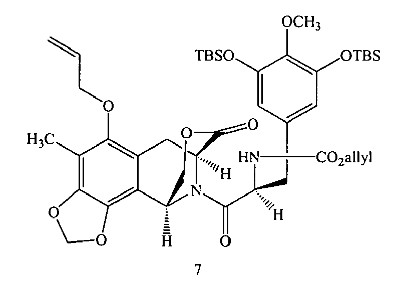
(c) réduction du composé lactone 7 en le lactol correspondant, composé 8 : 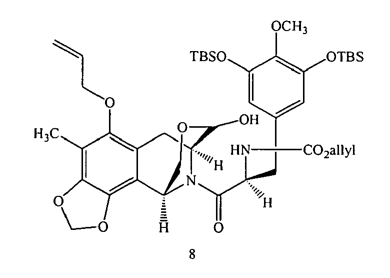
(d) désilylation du composé 8 en composé 9 : 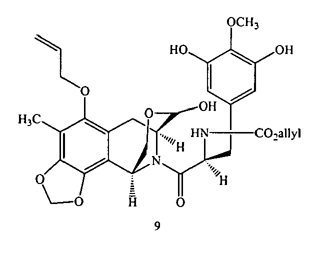
(e) cyclisation du composé 9 en composé 10 : 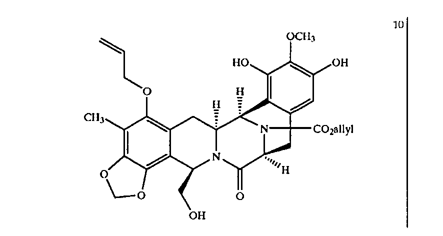
(f) réduction du lactam dans le composé 10 en l'aminal cyclique correspondant qui sur exposition au HCN a donné l'amino-nitrile pentacyclique, composé 5 :

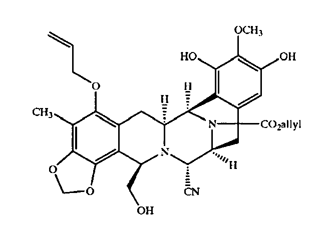

2. Le procédé de la revendication 1 pour préparer de la phtalascidine, comprenant en outre les étapes consistant en :
(g) la conversion du composé de la formule 5 en le composé de la formule 11 par traitement avec du PhNTf₂ 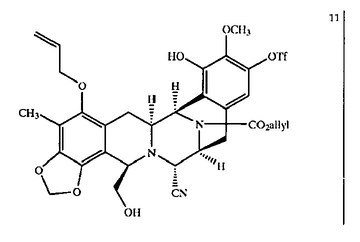
(h) la conversion du composé 11 en l'éther mono-t-butyldiméthylsilylique (TBS), composé 12 : 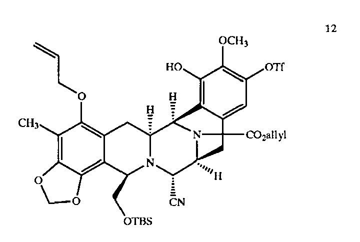
(i) éthérification avec du chlorure de méthoxyméthyle pour donner le composé 13 : 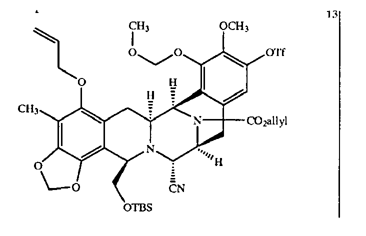
(j) le clivage des groupes N-allyloxycarbonyle et O-allyle dans 13 donne l'amine secondaire, composé 14 : 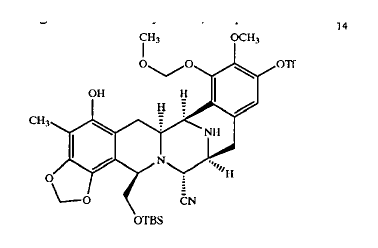
(k) la N-méthylation du composé 14 donne le composé 15 : 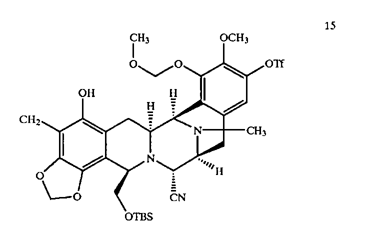
(l) la C-méthylation du composé 15 donne le composé 16 : 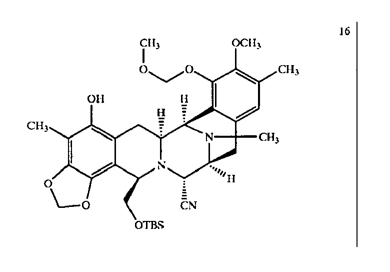
(m) l'acétylation du phénol 16 donne l'acétate correspondant, composé 17 : 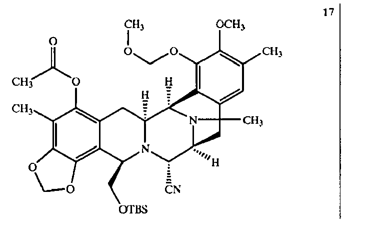
(n) la désilylation du composé 17 donne l'alcool primaire, composé 18 : 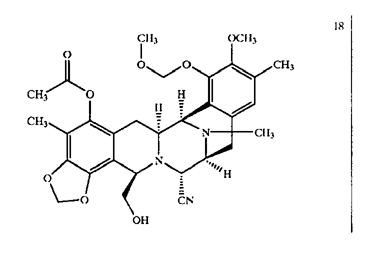
(o) le déplacement de Mitsunobu de l'hydroxyle primaire de 18, donne le phtalamide, composé 19 : 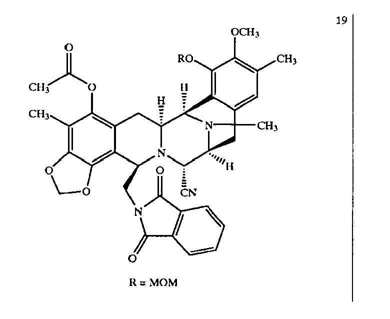
(p) le clivage catalysé par acide de l'éther méthoxyméthylique dans le composé 19, produit le composé 2 - phtalascidine :

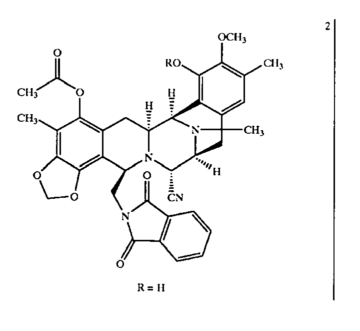

3. L'intermédiaire synthétique du procédé de la revendication 1 - composé 6 :

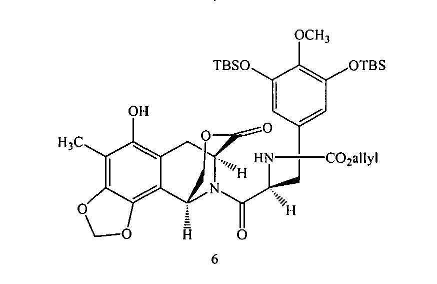

4. L'intermédiaire synthétique du procédé de la revendication 1 - composé 7 :

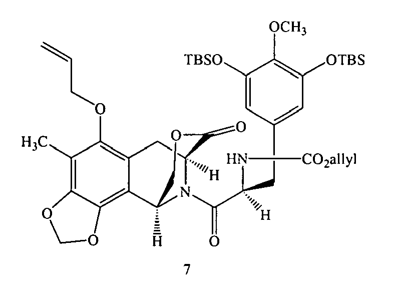

5. L'intermédiaire synthétique du procédé de la revendication 1 - composé 8 :

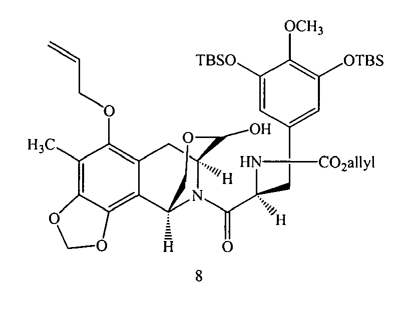

6. L'intermédiaire synthétique du procédé de la revendication 1 - composé 9 :

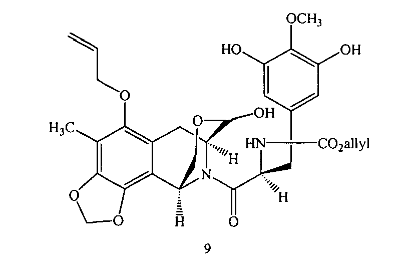

7. L'intermédiaire synthétique du procédé de la revendication 1 - composé 10 :

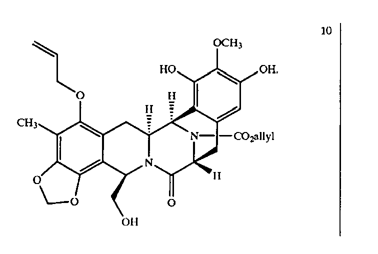

8. L'intermédiaire synthétique du procédé de la revendication 2 - composé 12 :

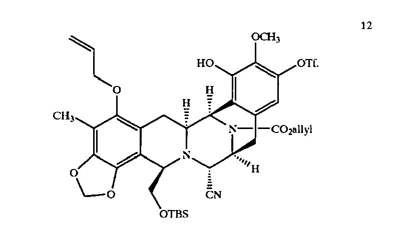

9. L'intermédiaire synthétique du procédé de la revendication 2 - composé 13 :

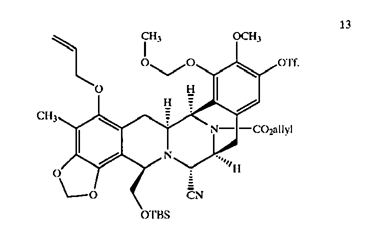

10. L'intermédiaire synthétique du procédé de la revendication 2 - composé 14 :

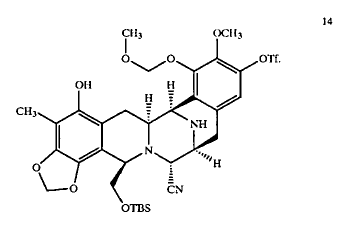

11. L'intermédiaire synthétique du procédé de la revendication 2 - composé 15 :

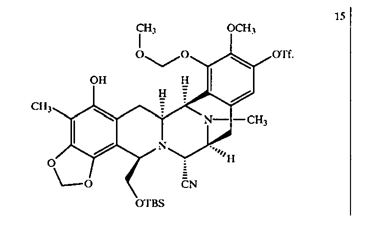

12. L'intermédiaire synthétique du procédé de la revendication 2 - composé 16 :

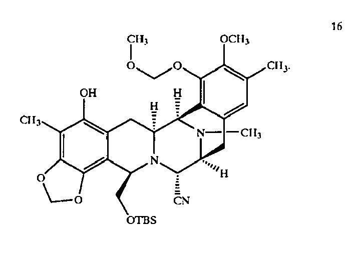

13. L'intermédiaire synthétique du procédé de la revendication 2 - composé 17 :

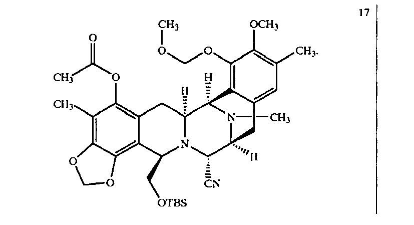

14. L'intermédiaire synthétique du procédé de la revendication 2 - composé 18 :

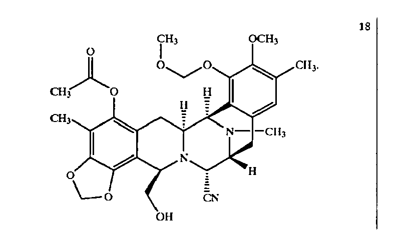

15. L'intermédiaire synthétique du procédé de la revendication 2 - composé 19:
